Europäisches Patentamt

European Patent Office

Office européen des brevets

(11) Publication number: **0 463 508 A1**

# EUROPEAN PATENT APPLICATION

(21) Application number: **91109774.9**

(22) Date of filing: **14.06.91**

(51) Int. Cl.⁵: **C12N 15/89**, C12N 5/00

(30) Priority: **29.06.90 JP 174305/90**

(43) Date of publication of application:
**02.01.92 Bulletin 92/01**

(84) Designated Contracting States:
**DE FR GB IT**

(71) Applicant: **W.R. Grace & Co.-Conn.**
**Grace Plaza, 1114 Ave. of the Americas**
**New York New York 10036(US)**

(72) Inventor: **Mori, Yuichi**
**1642-212-B-4 Kamariva-cho**
**Kanagawa-ken(JP)**
Inventor: **Yamazaki, Manabu**
**Corpo. Tsurumaki A-405, 1200-1 Tsurumaki**
**Hadano-shi, Kanagawa-ken(JP)**
Inventor: **Takezawa, Toshiaki**
**Nifty 34-302**
**31-15 Higashinaruse, Isehara-shi(JP)**

(74) Representative: **UEXKÜLL & STOLBERG**
**Patentanwälte**
**Beselerstrasse 4**
**W-2000 Hamburg 52(DE)**

(54) **Method for injecting substances into cells.**

(57) The present invention provides a method for introducing a substance into a cell on a plate capable of immobilizing and easily detaching the cells. More specifically, the method employs the use of a temperature-responsive polymeric compound and a cell adhesive substance to immobilize and detach the cells.

EP 0 463 508 A1

## Technical Field

The present invention relates to a method for injecting a substance, such as a gene, into cells on a plate capable of immobilizing and detaching the cells. The method can be conveniently and effectively used in the fields of genetic engineering, protein engineering, cell engineering, embryonic engineering and tissue/organ engineering without causing any damage to the cells.

## Background Art

A variety of methods have been developed for transferring substances such as genes into cells. These methods include chemical methods using calcium phosphate, DEAE dextran, etc., protoplast fusion methods, mechanical methods such as microinjection, pricking, laser bombardment, particle bombardment or electroporation methods. As compared to other methods, the mechanical methods, such as microinjection, demonstrate the most efficient gene transferring activity as shown by high expression frequency of transformants. High transformant expression frequency is an important characteristic of the microinjection method, particularly when the amount of cells obtained from a living body is extremely small, e.g., in the case of fertilized eggs obtained from the oviduct or uterus of a living body for the preparation of transgenic animals. Another benefit of the microinjection method is the ability to selectively inject a gene only into a targeted cell and to simply control the amount of transferred genes.

However, the mechanical method has significant problems as follows. In order to inject genes into cells, the immobilization of the cells is absolutely necessary. The immobilization method most commonly used in the microinjection method is to allow the tip of a glass pipette (diameter of the tip is 50 to 100 $\mu$m) to be in contact with the cell membrane and negative pressure to be created in the pipette during the procedure of injection of genes into the cell using a glass microcapillary pipette being 1 to 2 $\mu$m in diameter at the tip. These procedures are carried out under a microscope. This immobilization method requires not only a very skillful technique but is also very time consuming since each cell must be fixed individually. The possibility of causing serious physical damage to the cell membrane by attaching the glass pipette to the cell under negative pressure is high.

In order to solve the problems associated with fixing cells for the microinjection method, an immobilization method using a cell-fixing plate in which cells adhere was developed, eliminating the use of a glass pipette. This method was much simpler than the immobilization method using a glass pipette and also reduced the physical damage of the cell membrane caused by the glass pipette, but it has been found that this method has a serious problem. For the recovery of the cells which have received the gene transfer treatment, cell detaching agents such proteolytic enzymes (i.e., trypsin) and EDTA are absolutely necessary to detach the cells from the cell-fixing plate. It is well known that these cell detaching agents are very harmful to cellular functions because they severely digest and destroy the cell membrane and membrane-bound receptors.

The present invention provides a method for injecting a substance, such as a gene, into cells on a plate capable of immobilizing and detaching cells without the above described problems.

## Definition

The term "LCST" is used herein to mean a lower critical solution temperature which is a transition temperature of a temperature-responsive polymeric compound between hydration and dehydration.

## Summary of the Invention

A method for the injection of a substance to a cell in accordance with the present invention comprises the steps of:

(i) immobilizing a cell on a plate comprising
   (a) a temperature-responsive polymeric compound having an LCST lower than the injection temperature and
   (b) a cell adhesive substance,
at a temperature higher than said LCST,
(ii) injecting a substance into the cell immobilized on said plate at a temperature higher than said LCST, and
(iii) lowering said temperature to a temperature below said LCST to detach and recover the cells injected with the substance.

## Detailed Description of the Invention

According to the present invention, substances such as genes can be injected into cells using a plate comprising a temperature-responsive polymeric compound having an LCST lower than the injection temperature and a cell adhesive substance capable of effectively immobilizing cells. It is possible to immobilize the cells onto the plate for the injection or introduction of the substances into cells at a temperature higher than the LCST and also detach the injected cells from the plate at a temperature lower than the LCST. Accordingly, immobilization of cells for the injection of the substances and recovery of the injected cells are conveniently and effectively performed merely by changing the temperature across the LCST. In the present invention one component of the plate, i.e., the cell adhesive substance is responsible for cell immobilization, and the other component, i.e. the temperature-responsive polymeric compound having an LCST is responsible for cell recovery. Thus, the combination of the above described two components is essential for the plate and the injection method of the present invention.

The temperature-responsive polymeric compound having an LCST lower than the injection temperature of this invention is in a solid state at the injection temperature. When the temperature is lowered to a temperature below the LCST, the temperature-responsive polymeric compound becomes soluble in the culture medium and the cells on the plate detach therefrom.

Examples of suitable temperature-responsive polymeric compounds having an LCST lower than the injection temperature which can be used in the present invention are poly-N-substituted (meth)-acrylamide derivatives and their copolymers, polymethylvinylether, polyethylene oxide, etherized methylcellulose, and partially acetylated polyvinyl alcohol. Of these preferred compounds, more preferred are poly-N-substituted acrylamide derivatives, poly-N-substituted methacrylamide derivatives and their copolymers.

Preferred examples of such temperature-responsive polymeric compounds in the present invention are listed below, but this invention is not limited to these examples. The LCSTs of these polymers rise with the sequence of polymers listed below.

Poly-N-acryloyl piperidine, poly-N-n-propyl methacrylamide, poly-N-isopropyl acrylamide, poly-N,N-diethyl acrylamide, poly-N-isopropyl methacrylamide, poly-N-cyclopropyl acrylamide, poly-N-acryloylpyrrolidine, poly-N,N-ethylmethyl acrylamide, poly-N-cyclopropyl methacrylamide, poly-N-ethyl acrylamide.

The above described polymers may be homopolymers or copolymers with other monomers. Any hydrophilic monomers or hydrophobic monomers can be used as the monomers for copolymerization. Generally speaking, copolymerization with a hydrophilic monomer will raise the LCST, and copolymerization with a hydrophobic monomer will lower the LCST. With a proper selection of monomers, a copolymer with a desired LCST can be achieved.

Examples of suitable hydrophilic monomers are N-vinylpyrrolidone, vinylpyridine, acrylamide, methacrylamide, N-methyl acrylamide, hydroxyethyl methacrylate, hydroxyethyl acrylate, hydroxymethyl methacrylate, hydroxymethyl acrylate, acrylic acid, methacrylic acid, vinyl sulfonic acid, styrylsulfonic acid their salts and N,N-dimethylaminoethyl methacrylate, N,N-diethylaminoethyl methacrylate, N,N-dimethylaminopropyl acrylamide and their salts, but the present invention is not limited to these compounds.

Examples of suitable hydrophobic monomers are acrylate derivatives and methacrylate derivatives such as ethyl acrylate, methyl methacrylate and glycidyl methacrylate; N-substituted alkyl (meth)acrylamide derivatives such as N-n-butyl (meth)acrylamide; vinyl chloride, acrylonitrile, styrene and vinyl acetate but the present invention is not limited to these compounds.

The molecular weight of the temperature- responsive polymeric compound which can be employed in the present invention is preferably at least about $1.0 \times 10^5$ and more preferably higher than about $1.0 \times 10^6$. The molecular weight herein means a number average molecular weight obtained from the viscosity. For example, the relationship between the number average molecular weight ($\overline{M}n$) of poly-N-isopropyl acrylamide and its intrinsic viscosity [$\eta$] can be represented by the following equation [S. Ito and R. T. Geronimo, Sen'i Kobunshi Zairyo Kenkyusho Hokoku, No. 159, p. 23 (1988)];

$$[\eta] = 9.59 \times 10^{-5}\ \overline{M}n^{0.65}$$

(in tetrahydrofuran solution at 27° C)

For example, poly-N-isopropyl acrylamide is a polymeric compound which shows a negative temperature coefficient of solubility in water (M. Heskins and J. E. Guillet, J. Macromol. Sci. Chem., A2(8), 1441, 1968). The hydrate (oxonium hydroxide) which depends on the hydrogen bonding formed at a lower temperature between a water molecule and the polymer molecule will decompose at a higher temperature, so that the polymer aggregates by dehydration to form a precipitate. Thus, the transition temperature of this hydration and dehydration is called "lower critical solution temperature" or "LCST". Thus, at a temperature above the LCST, the polymer aggregates to form a solid state, but at a temperature lower than the LCST, the polymer dissolves in water.

The present invention takes advantage of such properties of the temperature-responsive polymeric compound to allow cells to adhere onto the plate at a temperature higher than the LCST for the injection of a substance such as genes into cells and to detach the injected cells from the plate by lowering the temperature to a temperature below the LCST for recovery of the cells.

A serious problem associated with a plate comprising only the temperature-responsive polymeric compound is very poor cell attachability thereto and it is impossible to immobilize cells onto the plate for injection. Accordingly, in order to use the temperature-responsive polymeric compound for the plate, the combination of the above described other component, i.e., a cell adhesive substance capable of effectively immobilizing cells onto the plate is essential.

In the present invention such cell adhesive substances include extracellular matrix components, gelatin, lectins, anchorage oligopeptides which are the binding sites of anchorage proteins such as fibronectin, adhesive proteins isolated from shellfish and positively charged polymers. The extracellular matrix components are the substances existing among cells within a living body and include collagen, fibronectin, laminin, vitronectin, proteoglycan, glycosaminoglycan and thrombospondin. Examples of such positively charged polymers include polylysin, polyhistidine, protamine sulfate, polydimethylaminoethyl acrylate or methacrylate, polydiethylaminoethyl acrylate or methacrylate, polydimethylaminopropyl acrylate or methacrylate, polyethyleneimine and polyvinylpyridine.

The present invention has perfected a method wherein cells can be anchored for the injection of substances and then the cells can be detached for recovery by using the combination of the above described temperature-responsive polymeric compound and the above described cell adhesive substance.

A substance can be injected or introduced into a cell by a number of methods known in the art. The preferred methods are mechanical methods such as microinjection, pricking, laser or particle bombardment or electroporation. The most preferred method is microinjection.

The present invention has perfected a method of injecting a substance to a cell at a temperature higher than the LCST and recovering the injected cells by lowering the temperature to a temperature below the LCST by using the above described method.

The plate used for the method of the present invention typically consists of a coating of the temperature-responsive polymeric compound having an LCST lower than the injection temperature and the cell adhesive substance formed on a supporting material.

Such a plate is prepared by coating an aqueous solution of a mixture of the temperature-responsive polymeric compound and the cell adhesive substance on all or part of the surface of the supporting material at a temperature below the LCST and drying the coating thus obtained. The plate can also be prepared by dipping the supporting material into an aqueous mixture solution of the temperature-responsive polymeric compound and the cell adhesive substance at a temperature below the LCST and drying the coating thus obtained.

The mixing weight ratio of the temperature-responsive polymeric compound to the cell adhesive substance which can be employed in the present invention is typically from about 1:0.01 to about 1:3. This ratio varies depending on the type of cell adhesive substance used.

The thickness of the coating after it is dried is at least about 0.2 $\mu$m, preferably at least 0.5 $\mu$m and more preferably at least about 1.0 $\mu$m. When the thickness is below about 0.2 $\mu$m, the cell detachability remarkably worsens and it takes a very long time for the detachment of the cells and as a result, the cell functions are rendered unstable.

Further, the plate used in the present invention can be prepared by forming a layer of the temperature-responsive polymeric compound on a supporting material and then forming a layer of the cell adhesive substance on the layer of the temperature-responsive polymeric compound. Also, the plate used in the present invention can be prepared by forming a layer of the cell adhesive substance on a supporting material and then forming a layer of the temperature-responsive polymeric compound on the layer of cell adhesive substance.

The supporting material which can be employed in the present invention is preferably transparent or translucent, and is preferably of glass or of plastic. Exemplary plastics include polystyrene, polycarbonate, polymethyl methacrylate, polypropylene, polyethylene, polyester, polyamide, polyvinylidene fluoride, polyoxymethylene, polyvinylchloride, polyacrylonitrile, polytetrafluorethylene and polydimethylsiloxane.

There is no particular limitation on the shape of the supporting material, and it can take various shapes such as a dish, plate, film or sheet.

Both plant and animal cells can be employed in the present invention; however, the preferred cells are mammalian cells such as tissue and/or organ cells, blood cells and oocytes.

The substances which can be employed in the present invention include genes, embryos, proteins, mRNAs, plasmid vectors, enzymes and viruses.

The examples which follow are given for illustrative purposes and are not meant to limit the invention described herein.

Example 1

N-isopropyl acrylamide monomer (herein"NIPAAm", a product of Eastman Kodak Co.) 50 g was dissolved in benzene 500 ml, and 2,2'-azobisisobutyronitrile 0.2 g was used as the polymerization initiator to conduct polymerization at 60 °C for 12 hours in a stream of nitrogen gas with constant agitation. The polymer precipitated in benzene, was decanted, and the precipitate was dissolved in tetrahydrofuran and then purified by precipitation with ethyl ether to obtain poly-N-isopropyl acrylamide (herein "PNIPAAm"). The PNIPAAm thus prepared had a number average molecular weight of $2.0 \times 10^6$, and the LCST of the 0.5% (w/v) aqueous PNIPAAm solution and the LCST of the 1.0% (w/v) PNIPAAm phosphate buffer solution (PBS) were determined by turbidimetry, and they were about $32°C$ and about $29°C$, respectively. After sterilizing the 0.5% (w/v) aqueous PNIPAAm solution by filtration through a 0.45 $\mu$m filter, the aqueous polymer solution was mixed with an equal volume of the 0.5% (w/v) aqueous type I collagen solution derived from pepsinized calf skin (sterilized, a product of Kohken K.K.) to prepare a mixed solution containing 0.25% (w/v) PNIPAAm and 0.25% (w/v) collagen as the final concentrations. About 400 $\mu$l of this mixed solution was poured onto the bottom of a commercial tissue culture plastic dish (Falcon, 35 mm), and it was air-dried aseptically at about $10°C$ in a clean bench. A dish coated with a mixture of collagen and PNIPAAm (1:1) to a thickness of about 2 $\mu$m was obtained by the above described method.

Oocytes were taken from an oviduct of female mice under the microscope into a culture fluid containing hyaluronidase, maintained there for several minutes, washed 2 to 3 times with the culture medium and then incubated in a $CO_2$ incubator at $37°C$. The coated dish as obtained above was set in a microinjection apparatus (IMT2-SYF, OLYMPUS K.K.) which was kept at $37°C$. When the temperature at the bottom of the dish reached $37°C$, the culture medium containing the oocytes kept at $37°C$ was poured onto the coated dish, and they were left to stand under this condition for 20 minutes. After confirming that the oocytes had firmly anchored and fixed to the bottom of the dish by mildly shaking the dish, about 5 pl of PBS was injected into the interior of the oocytes using a glass microcapillary pipette designed for the injection of DNA and having 1 to 2 $\mu$m in diameter at its tip. Successful injection of PBS into the oocytes was confirmed by swelling of the oocytes. After finishing the above described procedure, the dish containing the anchored oocytes injected with PBS was taken out of the microinjection apparatus, and the outside of the dish was cooled to about $10°C$ and kept at this temperature for about 15 minutes. The oocytes were released from the bottom surface of the dish and floated or suspended in the culture medium.

It was possible to inject into the cells without holding the oocytes by using a holding pipette, and thus the method of the present invention drastically reduced the work and difficulty of the conventional injection procedure. On the other hand, when the oocytes were fixed by a glass holding pipette according to the method of fixing the oocytes as described in the prior art, mechanical damage to the cell membrane was confirmed by the scanning electron microscope examination of the oocytes after holding with the pipette. In contrast, no morphological abnormality was observed with the oocytes which were fixed and released according to the method of the present invention. Thus, the developing rate of transgenic animals is expected to remarkably be increased in the present invention.

Comparative Example 1

Using the same 0.5% (w/v) aqueous type I collagen solution as used in Example 1 the dish coated with only PNIPAAm to a thickness of about 2 $\mu$m was prepared by the same method as in Example 1. The culture medium containing the oocytes obtained by the same method as used in Example 1 was poured into the coated dish and was kept at about $37°C$ for about 60 minutes. However, the oocytes could not adhere and fix to the bottom surface of the coated dish. Accordingly, injection into the oocytes was impossible.

Comparative Example 2

Using the same 0.5% (w/v) aqueous type I collagen solution as used in Example 1, a dish coated with only collagen to a thickness of about 2 $\mu$m was prepared by the same method as in Example 1. The culture medium containing the oocytes obtained by the same method as used in Example 1 was poured into the coated dish and was kept at about $37°C$ for about 30 minutes. After confirming that the oocytes had firmly anchored and fixed to the bottom of the collagen coated dish by mildly shaking the dish, it was possible to inject PBS into the oocytes using the same method as used in Example 1. After the above described procedure, however, the oocytes injected with PBS could not be released from the dish by cooling the dish to about $10°C$ for one hour. Thus, the recovery of the injected oocytes was impossible.

Example 2

A mixed solution of concanavalin A (Con A, a product of Hohnen Seiyu K.K.), a type of lectin, with the aqueous PNIPAAm solution as used in Example 1 was prepared aseptically. This solution was poured onto the bottom of the dish similar to the one as used in Example 1 and dried to prepare a dish where a layer of the mixture of PNIPAAm and Con A (mixing weight ratio: 2:1) was coated to a thickness of about 0.9 $\mu$m. Two milliliters of a 37°C suspension solution of human dermal fibroblasts having a cell density of about $10^3$/ml was poured onto the coated dish and allowed to stand under this condition for one hour. After confirming that the cells had firmly anchored to the bottom of the dish by mildly shaking the dish, PBS was injected into the fibroblasts by the same method as used in Example 1. Successful injection into the fibroblasts without the use of holding pipette was confirmed by swelling of the fibroblasts. After injection, the coated dish was taken out of the microinjection apparatus and cooled to about 10°C for about 20 minutes. The fibroblasts spontaneously detached from the bottom surface of the coated dish and suspended in the culture medium. Thus easy recovery of the injected fibroblasts was possible.

Example 3

Using a mixed solution of the aqueous solution of polylysin (a product of Sigma Co.) with the same aqueous PNIPAAm solution as used in Example 1, a dish coated with a mixture of polylysin and PNIPAAm (mixing weight ratio 1:2) to a thickness of about 1 $\mu$m was prepared by the same method as used in Example 1. The oocytes as used in Example 1 could adhere onto the coated dish for the injection into the oocytes at 37°C, higher than the LCST, and then detach from the coated dish for recovery by lowering the temperature to about 10°C which was lower than the LCST.

**Claims**

1. A method for injecting a substance into a cell which comprises the steps of:
   (i) immobilizing a cell on a plate comprising
   (a) a temperature-responsive polymeric compound having an LCST lower than the injection temperature and
   (b) a cell adhesive substance, at a temperature higher than said LCST,
   (ii) injecting a substance into the cell immobilized on said plate at a temperature higher than said LCST, and
   (iii) lowering said temperature to a temperature below said LCST to detach and recover the cell injected with said substance from said plate.

2. The method of Claim 1 wherein said substance is injected by microinjection.

3. The method of Claim 1, wherein said temperature- responsive polymeric compound is selected from a group consisting of poly-N-substituted acrylamide derivatives, poly-N-substituted methacrylamide derivatives or their copolymers, polyvinyl methyl ethers and partially acetylated polyvinyl alcohols.

4. The method of Claim 1, wherein said cell adhesive substance is selected from a group consisting of extracellular matrix components, gelatin, lectins, anchorage oligopeptides, adhesive proteins isolated from shellfish, positively charged polymers and their mixtures.

5. The method of Claim 4, wherein said extracellular matrix component is selected from the group consisting of collagen, fibronectin, vitronectin, laminin, proteoglycan, glycosaminoglycan and thrombospondin.

6. The method of Claim 1, wherein said cell is a mammalian cell.

7. The method of Claim 6 wherein said mammalian cell is selected from the group consisting of tissue cells, organ cells, blood cells and oocytes.

8. The method of Claim 1, wherein said substance is selected from the group consisting of genes, embryos, proteins, mRNAs, plasmid vectors, enzymes and viruses.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-387 975 (W.R. GRACE & CO.CONN.)<br>* the whole document *<br>--- | 1,3-7 | C12N15/89<br>C12N5/00 |
| Y,P | EP-A-382 214 (KAO CORPORATION)<br>* the whole document *<br>--- | 1-7 | |
| Y | EP-A-350 714 (BIO-POLYMERS INC.)<br>* the whole document *<br>--- | 1-7 | |
| Y | EP-A-175 966 (CALGENE INC.)<br>* page 1, line 29 - line 34 *<br>* page 2, line 12 - line 31 *<br>* page 3, line 9 - line 14 *<br>* page 6, line 13 - page 7, line 10 *<br>* claim 1 *<br>--- | 1-7 | |
| Y | HOGAN, B ET AL. 'MANIPULATING THE MOUSE EMBRYO'<br>1986 , COLD SPRING HARBOR LABORATORY , NEW YORK, USA<br>* page 192 - page 195 *<br>--- | 1-7 | |
| A | GB-A-2 080 814 (CESKOSLOVENSKA AKADEMIE VED PRAHA CZECHOSLOVAKIA)<br>* claims 1,2 *<br>----- | 1 | TECHNICAL FIELDS SEARCHED (Int. Cl.5 )<br><br>C12N |

The present search report has been drawn up for all claims

| Place of search | Date of completion of the search | Examiner |
|---|---|---|
| THE HAGUE | 11 SEPTEMBER 1991 | CHAMBONNET F.J. |